# EUROPEAN PATENT SPECIFICATION

(11) **EP 3 389 637 B1**
(45) Date of publication and mention of the grant of the patent: **11.11.2020**
(21) Application number: 16876902.4
(22) Date of filing: 19.12.2016
(51) Int. Cl.: A61K 9/16, A61K 9/51, A61K 47/36, A61K 47/50, G01N 33/543

(54) **COMPOSITIONS FOR MODULATED RELEASE OF PROTEINS AND METHODS OF USE THEREOF**
ZUSAMMENSETZUNGEN ZUR MODULIERUNG DER PROTEINFREISETZUNG UND VERFAHREN ZUR VERWENDUNG DAVON
COMPOSITIONS POUR LIBÉRATION MODULÉE DE PROTÉINES ET LEURS MÉTHODES D'UTILISATION

(30) Priority: 17.12.2015 US 201562268588 P; 16.06.2016 US 201662350768 P
(43) Date of publication of application: 24.10.2018
(73) Proprietor: Board of Regents of the University of Nebraska, Lincoln, Nebraska 68583-0745 (US)
(72) Inventor: SOLHEIM, Joyce, Omaha NE 68124 (US); BRONICH, Tatiana, Elkhorn NE 68022 (US)
(74) Representative: Schneiter, Sorin
(86) International application number: PCT/US2016/067494
(87) International publication number: WO 2017/106827

(56) References cited:
- WO-A1-2011/150420
- WO-A1-2011/150420
- WO-A2-2004/096998
- US-A1- 2005 191 344
- US-A1- 2005 266 090
- US-B2- 8 592 364
- YANA WANG ET AL: "Engineering chemoattractant gradients using chemokine-releasing polysaccharide microspheres", BIOMATERIALS, ELSEVIER SCIENCE PUBLISHERS BV., BARKING, GB, vol. 32, no. 21, 10 March 2011 (2011-03-10), pages 4903-4913, XP028209385, ISSN: 0142-9612, DOI: 10.1016/J.BIOMATERIALS.2011.03.027 [retrieved on 2011-03-16]
- GANGULY, K ET AL.: 'Polysaccharide-based micro/nanohydrogels for delivering macromolecular therapeutics.' JOURNAL OF CONTROLLED RELEASE. vol. 193, 2014, pages 162 - 173, XP029084262
- KUZMOV, A ET AL.: 'Nanotechnology approaches for inhalation treatment of lung diseases.' JOURNAL OF CONTROLLED RELEASE. vol. 219, 19 August 2015, pages 500 - 518, XP029303642

## Description

This application claims priority under 35 U.S.C. §119(e) to U.S. Provisional Patent Application No. 62/268,588, filed December 17, 2015, and U.S. Provisional Patent Application No. 62/350,768, filed June 16, 2016.

This invention was made with government support under Grant No. RO3 CA173223 awarded by the National Institutes of Health. The government has certain rights in the invention.

### FIELD OF THE INVENTION

The present invention relates generally to the delivery of compounds. More specifically, the present invention relates to compositions and methods for the delivery of bioactive agents such as therapeutic agents to a patient, particularly for the treatment of a disease or disorder.

### BACKGROUND OF THE INVENTION

The immune system's antigen-presenting dendritic cells (DCs) are the major cells responsible for initiating an immune response against tumors, via presentation of antigens from engulfed tumor cells to T lymphocytes. The localization of DCs, as well as other immune cells, is determined by chemokines, and the chemokine CCL21 acts as a strong attractant for DCs, T cells, and natural killer (NK) cells (Robertson, M.J. (2002) J. Leukoc. Biol., 71:173-183; Cyster, J.G. (1999) Science 286:2098-2102; Willimann et al. (1998) Eur. J. Immunol., 28:2025-2034; Forster et al. (2008) Nature Rev. Immunol., 8:362-371). CCL21 injection can cause the development of immune cell foci with some resemblance to novel lymph nodes, which can prime T cell responses (Kirk et al. (2001) Cancer Res., 61:8794-8802; Ashour et al. (2007) Int. Immunopharmacol., 7:272-276; Dubinett et al. (2010) Cancer J., 16:325-335). In addition to its chemotactic function, CCL21 stimulates antigen uptake by DCs (Sanchez-Sanchez et al. (2004) Blood 104:619-625; Escribano et al. (2009) J. Immunol., 183:6282-6295; Yanagawa et al. (2003) Blood 101:4923-4929). One receptor for CCL21, called CCR7, is naturally expressed on naive T cells, central memory T cells, NK cells, and mature DCs (Yoshida et al. (1998) J. Biol. Chem., 273:7118-7122; Jenh et al. (1999) J. Immunol., 162:3765-3769; Sallusto et al. (1999) Nature 401:708-712; Serra et al. (2004) Allergy 59:1219-1223). Besides CCR7, the CCL21 chemokine also binds to another receptor, called CCRL1, which is expressed on lymphoid endothelial cells and which has recently has been shown regulate CCL21 bioactivity (Graham et al. (2012) Immunol. Lett., 145:30-38; Comerford et al. (2013) Cytokine Growth Factor Rev., 24:269-283; Ulvmar et al. (2014) Nature Immunol., 15:623-630). Yana Wang et al. (2011) Biomaterials, 32, 4903-4913; show alginate hydrogel microparticles (diameter 31 +/- 12 um) loaded with different chemokines including CCL21 used as carrier for controlled release of chemokines, and CaCl2 is present.

The CCL21 chemokine, when administered to pancreatic tumors, can attract anti-tumor T cells and DCs, and can reduce the growth of the tumors directly treated with CCL21 and also the growth of tumors at distant anatomic sites via CCL21's induction of systemic immunity (Turnquist et al. (2007) Int. J. Oncol., 30:631-639). Because of CCL21's small size (∼100 amino acids) and its relatively rapid degradation rate, approaches designed to prolong its presence at the tumor site must be developed to enable it to have maximal therapeutic effect.

### SUMMARY OF THE INVENTION

In accordance with the instant invention, nanoparticles are provided along with their methods of synthesis. The nanoparticles of the instant invention comprise alginate, C-C motif chemokine ligand 21(CCL21), protamine sulfate, and a divalent cation (e.g., Ca²⁺). The nanoparticles may further comprise an amphiphilic copolymer (e.g., a poloxamer). The CCL21 may be contained in a polymeric mesh (comprising the alginate, protamine sulfate, and/or amphiphilic copolymer) in the interior of the nanoparticle. In a particular embodiment, the nanoparticles of the instant invention have a diameter less than or equal to 300 nm. Compositions comprising the nanoparticles of the instant invention and a pharmaceutically acceptable carrier are also provided.

In accordance with another aspect of the instant disclosure, methods for treating, inhibiting, and/or preventing a disease or disorder in a subject are provided.

The scope of the invention is defined by the claims. Any references in the description to methods of treatment refer to the compounds, pharmaceutical compositions and medicaments of the present invention for use in a method for treatment of the human (or animal) body by therapy (or for diagnosis). In a particular embodiment, the disease or disorder is cancer. In a particular embodiment, the method comprises administering to the subject at least one nanoparticle of the instant invention. The methods may further comprise the administration of other therapeutic methods or compositions to the subject.

In accordance with another aspect of the instant invention, methods of synthesizing the nanoparticles of the instant invention are provided. In a particular embodiment, the method comprises a salt of the divalent cation to an aqueous solution of CCL21, alginate, and optionally, an amphiphilic copolymer, and adding protamine sulfate to the aqueous solution after addition of the salt, thereby generating the nanoparticles. The methods may further comprise isolating the synthesized nanoparticles.

### BRIEF DESCRIPTIONS OF THE DRAWING

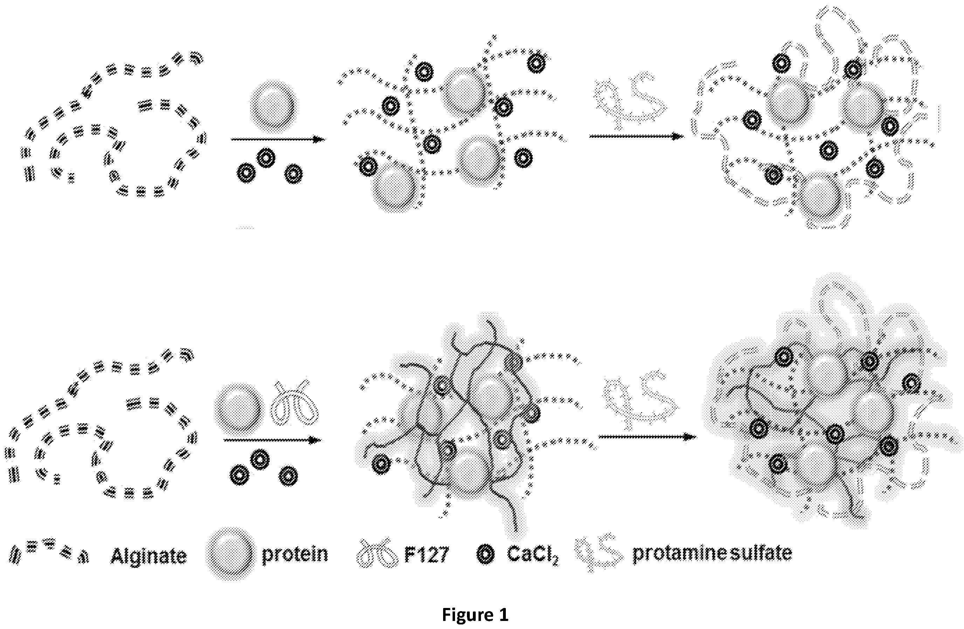

Figure 1 provides schematics of the formation of protein-loaded alginate-protamine sulfate nanoparticles (top) and composite alginate-Pluronic® F127 protamine sulfate nanoparticles (bottom).

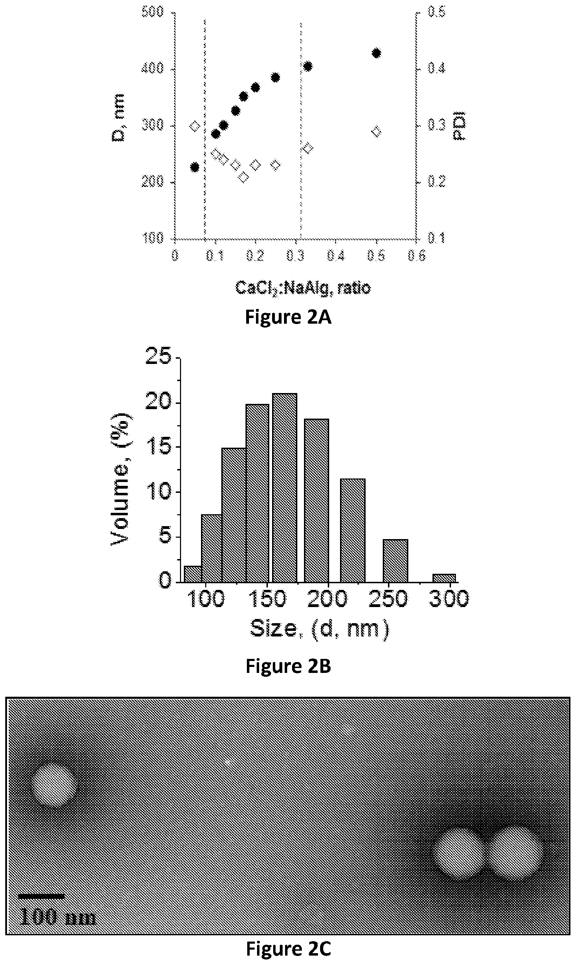

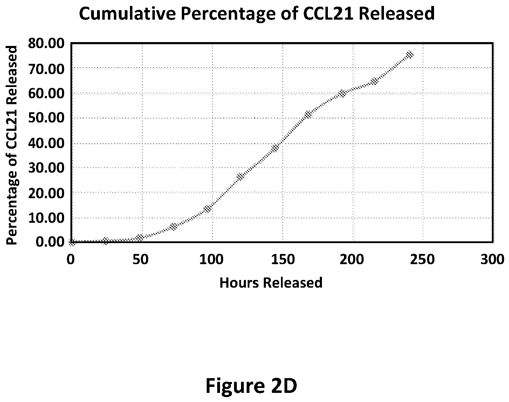

Figure 2A provides a graph of the diameter and polydispersity index (PDI) of CCL21-alginate nanoparticles based on varying the CaCl₂:alginate ratio. Diamonds: PDI. Black circles: diameter. Figure 2B provides a graph of the dynamic light scattering size distribution of CCL21-alginate nanoparticles comprising F127. Figure 2C provides a transmission electron micrograph of CCL21-alginate-F127 nanoparticles. Figure 2D provides a graph of the *in vitro* release of alginate:CCL21 (60:1) nanoparticles with F127. CCL21 was detected by ELISA.

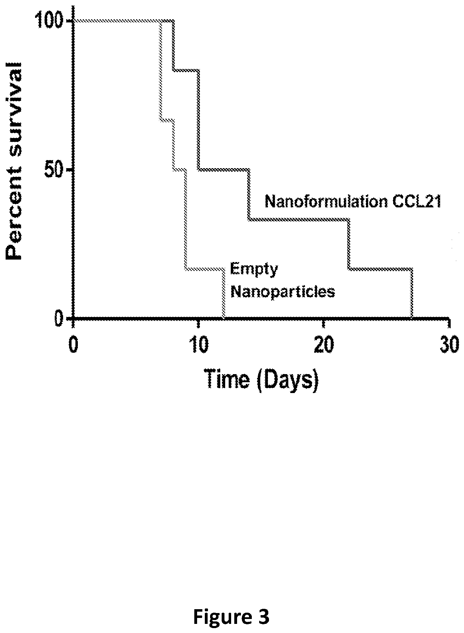

Figure 3 provides a Kaplan-Meier curve showing the percentage of A/J mice with subcutaneous Neuro2A neuroblastoma tumors surviving at various time points after intratumoral treatment with CCL21-alginate nanoparticles or empty control nanoparticles. For this experiment, the CCL21-alginate nanoparticle formulation included CaCl₂, protamine sulfate, and F127 in addition to murine CCL21 and alginate, whereas the empty nanoparticles lacked the CCL21. The mice were treated after the tumors were clearly palpable, and were euthanized when the tumor volumes were found to have reached or exceeded 1 cc at the time of monitoring. The median survival for nanoformulation CCL21-treated mice was 12 days and the median survival for the empty control nanoparticles-treated mice was 8.5 days (p=0.0276).

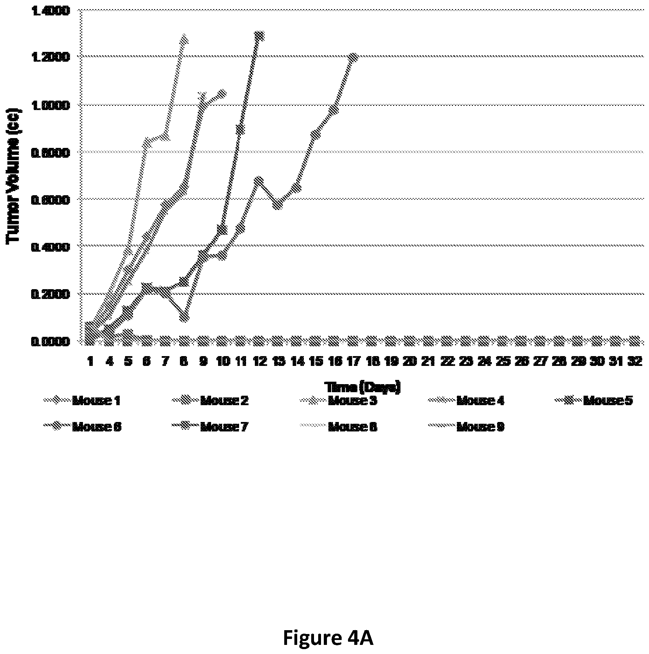

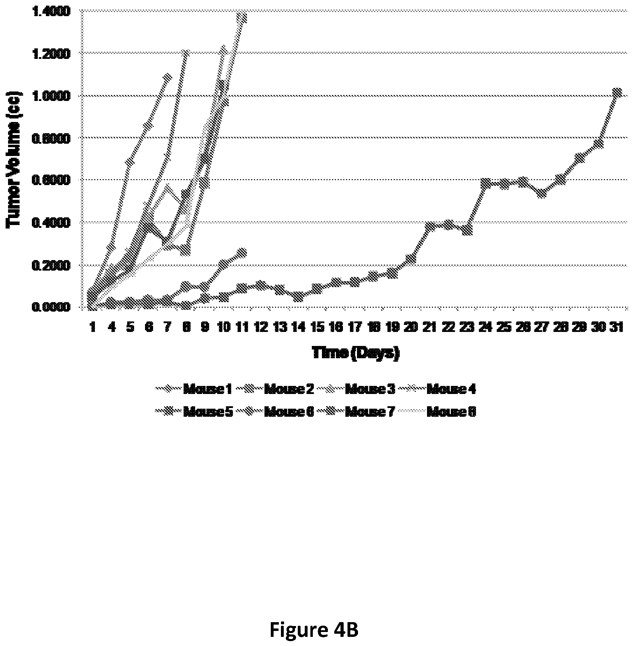

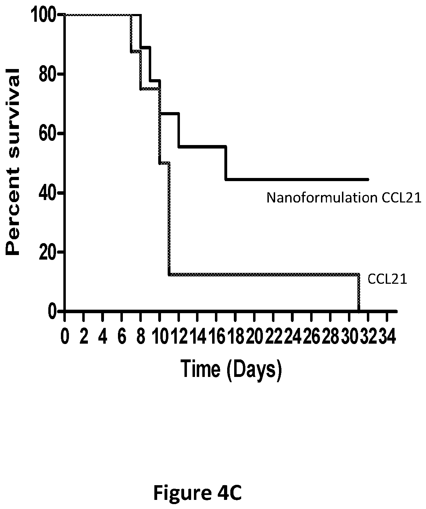

Figures 4A-4C illustrate data from a separate experiment in which A/J mice bearing palpable subcutaneous Neuro2A neuroblastoma tumors were treated with nanoformulation CCL21 or free CCL21 and were euthanized when the tumor volumes were found to have reached or exceeded 1 cc at the time of monitoring. The CCL21-alginate nanoparticle formulation included murine CCL21, alginate, CaCl₂, protamine sulfate, and F127. Figure 4A provides a graph of the change in tumor volume over time (through day 32) following treatment with alginate-CCL21 nanoformulation. Mouse 2, mouse 7, mouse 8, and mouse 9 had complete regression of their tumors. Figure 4B provides a graph of the change in tumor volume over time (through day 32) following treatment with free CCL21. Mouse 6 had to be euthanized due to tumor ulceration prior to the tumor reaching 1 cc in volume. Figure 4C provides a Kaplan-Meier curve of the survival of mice treated with CCL21 alone or CCL21 nanoparticles. The median survival for nanoparticle CCL21-treated mice was 17.0 days and the free CCL21-treated mice was only 10.5 days (p = 0.05).

### DETAILED DESCRIPTION OF THE INVENTION

The scope of the invention is defined by the claims. The present invention relates to a composition for the modulated release of proteins (inclusive of peptides), particularly biologically active proteins, for the treatment of a variety of diseases. In one embodiment the composition comprises a biocompatible polymeric matrix, a protein that is dispersed within the polymeric matrix, and, optionally, an amphiphilic polymer component which is separately dispersed within the polymeric matrix. In a particular embodiment the composition comprises a polyanion (e.g., alginate), a divalent cation (e.g., Ca²⁺ or a salt thereof (e.g., calcium chloride)) as a gelation agent, a polycation (e.g., protamine sulfate), the biologically active protein and, optionally, an amphiphilic polymer such as a poloxamer (e.g., poloxamer 407 (Pluronic® F127)). In a particular embodiment, the biologically active protein is chemokine CCL21, particularly human CCL21 (C-C motif chemokine ligand 21; see, e.g., GenBank Gene ID: 6366; GenBank Accession Nos: NP_002980 and NM_002989) or mouse CCL21 (see, e.g., GenBank Gene ID: 18829; GenBank Accession Nos: NM_011124.4 and NP_035254.1). In a particular embodiment, the biologically active protein (e.g., CCL21) is of the same species as the subject being treated (e.g., canine CCL21 for treating dogs, etc.). The incorporation of the protein into the polymer composite of the instant invention provides for strong retention in the carrier particles and protects the protein from premature release and degradation. Incorporation of amphiphilic copolymers into cross-linked alginate network further modifies the porosity and/or permeability of the alginate nanoparticles for tailoring the release of the protein.

Immunotherapies are viable options for targeting both the primary tumor and disseminated disease, and responses against tumors can be initiated by DCs via presentation of antigens from endocytosed tumor cells to T lymphocytes (Koido et al. (2012) Immunotherapy 4:5-7; Petersen et al. (2010) Crit. Rev. Immunol., 30:345-386). In murine models of melanoma, lung cancer, lymphoma, and colon cancer, intratumoral treatment with CCL21, which attracts DCs and T cells, as well as NK cells, has been shown to inhibit cancer growth (Kirk et al. (2001) Cancer Res., 61:2062-2070; Yang et al. (2004) Clin. Cancer Res., 10:2891-2901; Yang et al. (2006) Cancer Res., 66:3205-3213; Kar et al. (2011) PLoS One 6:e18758; Tolba et al. (2002) Cancer Res., 62:6545-6551). Intratumoral injection of CCL21 can increase immune infiltration and slow the growth of mammary tumors and subcutaneous pancreatic tumors (Jenh et al. (1999) J. Immunol., 162:3765-3769; Ashour et al. (2007) Cancer Biol. Ther., 6:1206-1210). CCL21 has entered clinical trials for lung cancer and melanoma (Dubinett et al. (2010) Cancer J., 16:325-335; Baratelli et al. (2008) J. Transl. Med., 6:38 (1-17); Sharma et al. (2013) Int. Trends Immun., 1:10-15; Mule, J.J. (2009) Cancer Vaccines 1174:33-40). One of the great benefits of CCL21 intratumoral therapy is that it provides personalized immunotherapy by using the patient's own *in vivo* tumor as the immunogen, but uses a single biologic agent (i.e., CCL21) that is not patient-specific.

Injection of CCL21 results in formation of lymphoid structures and attraction of DCs. The ectopic expression of CCL21 is capable of inducing the formation of structures composed of lymphocytes and DCs, which are able to prime antigen-specific T cell responses (Kirk et al. (2001) Cancer Res., 61:8794-8802; Ashour et al. (2007) Int. Immunopharmacol., 7:272-276; Dubinett et al. (2010) Cancer J., 16:325-335). Furthermore, when the effect of subcutaneously injected CCL21 on the frequency of DCs within the draining lymph node was analyzed at day 4 post-injection, a significant increase was found in the percentages of total draining lymph node cells that were DCs. In addition, a flow cytometric analysis of the cells infiltrating CCL21-treated subcutaneous Panc02 pancreatic tumors showed that intratumoral DCs were significantly increased relative to the numbers of DCs in PBS-treated tumor controls (Turnquist et al. (2007) Int. J. Oncol., 30:631-639).

CCL21 reduces the rate of tumor growth by an immunological mechanism. Following the injection of Panc02 pancreatic cancer cells and the development of subcutaneous tumors in C57BL/6 mice, intratumoral injections of CCL21 was initiated and the treatment was found to significantly inhibit the rate of tumor growth (Turnquist et al. (2007) Int. J. Oncol., 30:631-639). A significant decrease in growth was observed beginning on day 4 following the initial treatment (Turnquist et al. (2007) Int. J. Oncol., 30:631-639). The maximal inhibition was observed on days 11-14, at which time CCL21-treated tumors were 85% smaller than tumors treated with PBS (Turnquist et al. (2007) Int. J. Oncol., 30:631-639). Statistical analysis of tumor volumes from the treatment groups showed significant inhibition of tumor growth in the CCL21-treated group through day 18 (Turnquist et al. (2007) Int. J. Oncol., 30:631-639).

To confirm that the mechanism underlying CCL21's therapeutic activity in this model was immunological, CCL21 was also injected into subcutaneous Panc02 tumors in immunodeficient C57BL/6-RAG2^{-/-}Pfp^{-/-} mice, in conjunction with the studies in wild type C57BL/6 mentioned above (Turnquist et al. (2007) Int. J. Oncol., 30:631-639). RAG2^{-/-}Pfp^{-/-} mice have inactivated genes for RAG2 and perforin, and are therefore deficient in T and B cell development and in T/NK cell-mediated cytolytic killing. Comparison of the results on day 14 after the initiation of therapy for pancreatic tumors on C57BL/6 and C57BL/6 RAG2^{-/-}Pfp^{-/-} mice showed that CCL21-treated RAG2^{-/-}Pfp^{-/-} tumors were very similar in size to the tumors of PBS-treated wild type and RAG2^{-/-}Pfp^{-/-} mice (Turnquist et al. (2007) Int. J. Oncol., 30:631-639). In contrast, CCL21-treated tumors of immunocompetent C57BL/6 mice were ∼5-fold smaller than the tumors of the other groups (Turnquist et al. (2007) Int. J. Oncol., 30:631-639). CCL21 significantly reduced the time for tumors on the C57BL/6 mice to reach a volume of 1100 mm³ (median 16 days for the PBS group, versus 30 days for the CCL21 group) (Turnquist et al. (2007) Int. J. Oncol., 30:631-639). In contrast, a deceleration of growth following CCL21 treatment was not observed for the tumors of CCL21-treated RAG2^{-/-}Pfp^{-/-} mice (Turnquist et al. (2007) Int. J. Oncol., 30:631-639). Overall, these data indicate that CCL21's effect is mediated by immune cells, such as T cells and/or NK cells.

Intratumoral CCL21 treatment produces systemic anti-tumor activity. Subcutaneous tumors were established in both flanks of C57BL/6 mice by injection of Panc02 cells. Once palpable, the right flank tumors were treated with CCL21 (or PBS control), but the palpable left flank tumors of both groups received no injections. Intratumoral CCL21 injection significantly inhibited the growth of the directly injected tumors (Turnquist et al. (2007) Int. J. Oncol., 30:631-639). Moreover, inhibition was not limited to the treated tumors, since growth of the contralateral tumors (not injected with CCL21) was also significantly slowed (Turnquist et al. (2007) Int. J. Oncol., 30:631-639). Specifically, for mice that had received CCL21 in the right flank tumors, a 14-fold growth rate reduction was observed for the untreated left flank tumors through day 18 (Turnquist et al. (2007) Int. J. Oncol., 30:631-639). Untreated left flank tumors in the mice that were treated in the right flank tumors with CCL21 were consistently smaller, averaging 23 mm³ in volume on day 18, whereas the untreated left flank tumors in mice that had been treated with PBS in the right tumors had a mean volume of 354 mm³ on day 18 (Turnquist et al. (2007) Int. J. Oncol., 30:631-639). These data indicate that CCL21 treatment initiates immune responses that act in the local tumor environment and also against spatially distant tumors.

Using histochemical analysis, flow cytometry, and confocal microscopy, the immune cell infiltration of subcutaneous pancreatic tumors that had been injected with CCL21 (or PBS, as a control) were assessed (Turnquist et al. (2007) Int. J. Oncol., 30:631-639). Compared to control-treated tumors, CCL21-treated tumors had increased inflammatory cell infiltrates, and foci of leukocytes around tumor vasculature (Turnquist et al. (2007) Int. J. Oncol., 30:631-639). The CCL21-treated tumors had higher numbers of T cells (particularly CD8+ T cells), NK cells, NKT cells, and DCs (including DC subsets associated with anti-tumor responses, and not plasmacytoid DCs, which are associated with immunosuppression) (Turnquist et al. (2007) Int. J. Oncol., 30:631-639). A large majority of T cells within CCL21-treated tumors expressed low levels of CD62L, indicating they were activated T cells (Turnquist et al. (2007) Int. J. Oncol., 30:631-639).

Therefore, CCL21 is recognized as an advanced experimental biologic treatment for cancer. Though CCL21 is known to be successful as a tumor therapy, CCL21 is a very small and readily degraded protein. Therefore, maintaining it in slow release form within the tumor is important to increasing its effectiveness (Dubinett et al. (2010) Cancer J., 16:325-335; Baratelli et al. (2008) J. Transl. Med., 6:38 (1-17); Sharma et al. (2013) Int. Trends Immun., 1:10-15; Mule, J.J. (2009) Cancer Vaccines 1174:33-40; Kar et al. (2011) PLoS One 6:e18758; Wang et al. (2011) Biomaterials 32:4903-4913; Lee et al. (2013) Biomaterials 34:4860-4871).

Various methods of sustained CCL21 release have been tested either *in vitro* or *in vivo,* including transduced cells, vault nanocapsules, microspheres, and folic acid-upconversion nanoparticles (Dubinett et al. (2010) Cancer J., 16:325-335; Baratelli et al. (2008) J. Transl. Med., 6:38 (1-17); Sharma et al. (2013) Int. Trends Immun., 1:10-15; Mule, J.J. (2009) Cancer Vaccines 1174:33-40; Kar et al. (2011) PLoS One 6:e18758; Wang et al. (2011) Biomaterials 32:4903-4913; Lee et al. (2013) Biomaterials 34:4860-4871). Herein, the CCL21 nanoparticles of the instant invention possesses superior properties because they utilize formulations of materials that have a firmly established safety record, provide a desired release profile of the CCL21, protect the CCL21 from degradation, and are designed to be at a nanoscale level (e.g., an average diameter of ≤ 300 nm) to allow delivery by endoscopy. In contrast, other CCL21 nanodelivery systems that have been reported have undergone relatively limited testing in animals (Kar et al. (2011) PLoS One, 6:e18758; Lee et al. (2013) Biomaterials 34:4860-4871).

Herein, alginate/protamine sulfate nanoparticles have been produced in an aqueous environment, based on the ionotropic gelation of alginate with calcium chloride followed by crosslinking with protamine sulfate. Alginate is a natural, nontoxic, biodegradable material that is commercially available in a highly pure form and can be utilized for sustained release of proteins. Further, protamine sulfate has been previously established as a polycation with a good safety profile. The incorporation of protein into the polymer composite of the instant invention provides for retention of the protein in the nanoparticles and allows for slow release of the protein while protecting the protein from degradation. The incorporation of amphiphilic copolymers into the nanoparticles generally increases the retention of the protein within the nanoparticles.

The nanoparticles of the instant invention may be synthesized by alginate ionotropic pre-gelation in the presence of the biologically active protein (e.g., CCL21) followed by protamine sulfate polyelectrolyte complexation. To prepare composite nanoparticles, poloxamer (e.g., Pluronic® F127) (e.g., 0.5%) may be added to the alginate/protein solution prior to or with cross-linking with calcium ions. Incorporation of amphiphilic copolymers such as poloxamers into cross-linked alginate network can further alter the porosity/permeability of the alginate nanoparticles for tailoring the release of the biologically active protein. Initial formulation studies were done with cytochrome c (which has a similar size and charge to CCL21) before performing formulations with CCL21. The concentration of the gelation agent (e.g., calcium chloride), time of gelation process, concentration of protamine sulfate, and order of reagent addition may all be varied to prepare alginate particles in nanosize range (e.g., ≤ 300 nm), with low polydispersity, and high loading capacity. For example, as shown hereinbelow, lower CaCl₂ generally results in smaller alginate particles. However, at certain very low concentrations of CaCl₂, the polydispersity of the samples was found to increase and the particles were not as thermodynamically stable. CCL21 nanoparticles of the present invention can be formulated to have a desired diameter (e.g., ≤ about 300 nm), to be generally uniform (have a low polydispersity index (e.g., ≤ about 0.30, ≤ about 0.25 or ≤ about 0.20), have a spherical morphology, and exhibit high encapsulation efficacy of CCL21 (e.g., greater than about 90%). The release profile of such hybrid nanostructured polymeric materials can be controlled by selecting a polymer/or polymer mixture, by varying the type of amphiphilic polymer, and by adjusting parameters of the manufacturing process.

As explained above, the nanoparticles of the instant invention are designed to provide extended release of proteins (e.g., the chemokine CCL21) *in vivo,* allowing longer and greater therapeutic effectiveness. The nanosize range of the particles permits the delivery of biologically active materials via small gauge needles, which allows use of the nanoformulation in a variety of clinical applications. The nanoparticles of the instant invention were also determined to efficiently load the desired protein. Specifically, the efficiency of protein loading in the alginate nanoparticles was assessed by ELISA detection of free CCL21 in the supernatant. In a particular formulation, nanoparticles were prepared by mixing sodium alginate with CCL21 and stirring at room temperature for 10 minutes, adding CaCl₂ drop-wise at a ratio of 6:1 with stirring for an additional 30 minutes, and then adding protamine sulfate at 10:1, stirring at room temperature for 4 hours, and centrifuging. When the supernatant was collected and analyzed by ELISA for unloaded CCL21, the efficiency of loading was found to be very high (96.44%). Nanoparticles prepared by this approach were stable for at least a week when stored at 4°C. As shown hereinbelow in the Example, the CCL21-alginate nanoparticles of the instant invention were injected into subcutaneous Neuro2a neuroblastoma tumors in A/J mice and were able to decrease the tumor growth and to cause complete tumor regression in some mice, indicating that the CCL21 retained biological activity in the alginate nanoparticles.

In accordance with the instant invention, nanoparticles (sometimes referred to herein as nanoformulations) are provided. The nanoparticles of the instant invention may be used for the delivery of compounds, particularly proteins, to a subject or cell. In a particular embodiment, the nanoparticle comprises the protein dispersed with a polymeric matrix. In a particular embodiment, the nanoparticle comprises a polyanionic compound, a divalent cation, a polycationic compound, and a protein. The nanoparticles of the instant invention may further comprise an amphiphilic copolymer. The nanoparticles of the instant invention may also comprise a calcium phosphate (CaPO₄) shell. The components of the nanoparticles are discussed in more detail below.

In a particular embodiment, the nanoparticles of the instant invention have an average diameter of ≤ about 300 nm, ≤ about 275 nm, ≤ about 250 nm, or ≤ about 200 nm. In a particular embodiment, the nanoparticles of the instant invention have a polydispersity index of less than about 0.35, less than about 0.30, less than about 0.25, or less than about 0.20.

The polyanionic compound of the nanoparticles of the instant invention should be biocompatible and capable of forming a hydrogel with the protein, particularly in the presence of the divalent cation (e.g., undergo ionic gelation in the presence of divalent ions). In a particular embodiment, the polyanionic compound is an anionic polysaccharide. Examples polyanionic compounds include, without limitation: alginate, carragenn, pectin, and gum gellan. In a particular embodiment, the polyanionic compound is alginate.

The divalent cations of the nanoparticles of the instant invention can be, for example, Mg²⁺, Ca²⁺, Sr²⁺, or Ba²⁺. In a particular embodiment, the divalent cation is Ca²⁺. During the synthesis methods of the nanoparticles, the divalent cations may be provided in salt form. For example, a calcium salt such as CaCl₂ may be used to synthesize the nanoparticles of the instant invention.

The polycationic compound of the nanoparticles of the instant invention should be biocompatible and capable of forming nanoparticles with the anionic compound and protein containing hydrogel. Generally, any polycation can be used. Examples of cationic polymers include, but are not limited to: polymers and copolymers and their salts comprising units deriving from one or several monomers including: primary, secondary and tertiary amines, each of which can be partially or completely quaternized forming quaternary ammonium salts. Examples of these monomers include, without limitation: cationic amino acids (e.g., lysine, arginine, histidine), alkyleneimines (e.g., ethyleneimine, propyleneimine, butileneimine, pentyleneimine, hexyleneimine), spermine, vinyl monomers (e.g.,vinylcaprolactam, vinylpyridine), acrylates and methacrylates (e.g., N,N-dimethylaminoethyl acrylate, N,N-dimethylaminoethyl methacrylate, N,N-diethylaminoethyl acrylate, N,N-diethylaminoethyl methacrylate, t-butylaminoethyl methacrylate, acryloxyethyltrimethyl ammonium halide, acryloxyethyl-dimethylbenzyl ammonium halide, methacrylamidopropyltrimethyl ammonium halide), allyl monomers (e.g., dimethyl diallyl ammoniam chloride), aliphatic, heterocyclic or aromatic ionenes. In a particular embodiment, the polycationic compound is protamine sulfate.

The protein of the nanoparticles of the instant invention can be any protein to be delivered to the subject or cell. In a particular embodiment, the protein is a biologically active protein. In a particular embodiment, the protein is a cytokine, chemokine, or interleukin (e.g., a cytokine, chemokine, or interleukin which stimulates an immune response (e.g., recruits DCs, T cells, and/or natural killer (NK) cells); e.g., a proinflammatory cytokine, chemokine, or interleukin). In a particular embodiment, the protein is a small protein (or peptide) (e.g., less than about 25 kDa, less than about 20 kDa, or less than about 15 kDa). In a particular embodiment, the protein is a cytokine, particularly a chemokine (e.g., a homing cytokine or chemokine). In a particular embodiment, the protein is a ligand of CCR7. In a particular embodiment, the protein is CXCL10, CXCL12, CCL19, or CCL21, particularly CCL19 or CCL21. In a particular embodiment, the protein is CCL21.

The amphiphilic copolymer of the nanoparticles of the instant invention should be biocompatible and capable of forming nanoparticles with the other components of the nanoparticle. In a particular embodiment, the amphiphilic copolymer is an amphiphilic block copolymer. In a particular embodiment, the amphiphilic block copolymer is a diblock copolymer (e.g., A-B or B-A, wherein A is a hydrophilic block and B is a hydrophobic block) or a triblock copolymer (e.g., A-B-A or B-A-B, wherein A is a hydrophilic block and B is a hydrophobic block; particularly wherein the middle block is hydrophobic). In a particular embodiment, the segments of the amphiphilic block copolymer comprise about 20 to about 300 repeating units, about 20 to about 250 repeating units, about 20 to about 200 repeating units, about 20 to about 150 repeating units, about 20 to about 125 repeating units, or about 50 to about 125 repeating units. In a particular embodiment, the amphiphilic block copolymer comprises at least one block of poly(oxyethylene) and at least one block of poly(oxypropylene). In a particular embodiment, the amphiphilic block copolymer is a poloxamer. In a particular embodiment, the amphiphilic block copolymer is a triblock amphiphilic block copolymer comprising a central hydrophobic block of polypropylene glycol flanked by two hydrophilic blocks of polyethylene glycol. In a particular embodiment, the amphiphilic block copolymer is poloxamer 407.

The nanoparticles of the instant invention comprise alginate, a divalent cation, protamine sulfate, and a protein. The nanoparticles may further comprise an amphiphilic copolymer. In a particular embodiment, the divalent cation is Ca²⁺. In a particular embodiment, the protein is CCL21. In a particular embodiment, the amphiphilic copolymer is poloxamer 407. The nanoparticles of the instant invention may comprises alginate, Ca²⁺, protamine sulfate, CCL21, and, optionally, poloxamer 407. In a particular embodiment, the alginate:CCL21 ratio (by weight) of the nanoparticle is from about 0.1:1 to about 200:1, about 0.5:1 to about 150:1, about 1:1 to about 200:1, about 1:1 to about 100:1, about 1:1 to about 75:1, about 1:1 to about 60:1, about 2:1 to about 150:1, about 3:1 to about 100:1, about 5:1 to about 75:1, or about 6:1 to about 60:1. In a particular embodiment, the alginate: protamine sulfate ratio (by weight) in the nanoparticles is from about 0.1:1 to about 200:1, about 0.5:1 to about 150:1, about 1:1 to about 100:1, about 10:1 to about 100:1, about 1:1 to about 50:1, about 5:1 to about 50:1, about 10:1 to about 50:1, or about 3:1 to about 30:1. While these ratios are provided for alginate:CCL21, the same ratios may be used should the alginate and/or CCL21 be replaced with a different polyanionic compound or protein, respectively.

In accordance with another aspect of the instant invention, compositions comprising the nanoparticles are provided. In a particular embodiment, the composition comprises a nanoparticle of the instant invention and a pharmaceutically acceptable carrier. The compositions may further comprise at least one other therapeutic agent (e.g., chemotherapeutic agent, anti-cancer therapies, or immunotherapies (see below)).

In accordance with another aspect of the instant invention, methods are provided for synthesizing the nanoparticles of the instant invention. For simplicity, the method is described with the polyanionic compound as alginate, the polycationic compound as protamine sulfate, and the protein as CCL21. However, the same methods and ratios may be used should the alginate, protamine sulfate, and/or CCL21 be replaced with a different polyanionic compound, polycationic compound, or protein, respectively. In a particular embodiment, the method comprises adding the divalent cation, particularly in salt form (e.g., CaCl₂), to a solution (e.g., aqueous solution) of CCL21 and alginate (e.g., with agitation and/or mixing). The solution may further comprise the amphiphilic copolymer (e.g., poloxamer 407) prior to addition of the divalent cation. In a particular embodiment, the alginate:CCL21 ratio (by weight) in the solution is from 0.1:1 to about 200:1, about 0.5:1 to about 150:1, about 1:1 to about 200:1, about 1:1 to about 100:1, about 1:1 to about 75:1, about 1:1 to about 60:1, about 2:1 to about 150:1, about 3:1 to about 100:1, about 5:1 to about 75:1, or about 6:1 to about 60:1. In a particular embodiment, the amphiphilic copolymer is present in the solution from about 0.01% to about 10%, about 0.05% to about 5%, or about 0.1 to about 1.0%. In a particular embodiment, the divalent cation salt (e.g., CaCl₂)):alginate ratio (by weight) is about 0.01 to about 2.0, about 0.05 to about 1.0, about 0.05 to about 0.75, about 0.05 to about 0.50, about 0.075 to about 0.50, or about 0.1 to about 0.50.

The method further comprises adding protamine sulfate to the above aqueous solution (e.g., with agitation and/or mixing) in order to generate the naoparticles. In a particular embodiment, the alginate: protamine sulfate ratio (by weight) in the solution is from 0.1:1 to about 200:1, about 0.5:1 to about 150:1, about 1:1 to about 100:1, about 10:1 to about 100:1, about 1:1 to about 50:1, about 5:1 to about 50:1, about 10:1 to about 50:1, or about 3:1 to about 30:1. The methods may further comprise isolating the nanoparticles (e.g., by centrifugation).

The nanoparticles of the present invention (or compositions comprising the nanoparticles) can be used for the treatment of a variety of diseases including, but not limited to, cancer, infectious diseases, autoimmune disorders (e.g., at toleragenic dosages) and transplant rejection (e.g., organ transplant). In a particular embodiment, the disease is cancer. Cancers that may be treated using the nanoparticles of the present invention include, but are not limited to: melanoma (multiple myeloma), lung cancer, lymphoma, leukemia, colon cancer, breast cancer, neuroblastoma, pancreatic cancer, prostate cancer, liver cancer, kidney cancer, brain cancer, thyroid cancer, bladder cancer, bone cancer, stomach cancer, esophageal cancer, leukemia, multiple myeloma, sarcoma, carcinoid, skin cancer, testicular cancer, ovarian cancer, pancreatic cancer, colorectal cancer, cervical cancer, endometrial cancer, or renal cancer. In a particular embodiment, the cancer forms a solid tumor. In a particular embodiment, the cancer is neuroblastoma. The nanoparticles may be delivered locally to the site of the disease (e.g., tumor (e.g., intratumorally)). For example, the nanoparticles may be delivered via small gauge needles, including but not limited to those used for endoscopic ultrasound-guided injections. Therefore, biological agents can be delivered to tissues (such as tumors) in a sustained, continuous and predictable manner. Since the biological agent can be administered locally, systemic toxicity is minimized.

Thus, according to another aspect of the instant invention, methods for treating, inhibiting, and/or preventing a disease or disorder in a subject are provided. In a particular embodiment, the disease is, without limitation: cancer, infectious diseases (e.g., disease caused by pathogenic microorganisms such as bacteria, viruses, parasites or fungi), autoimmune disorders (e.g., at toleragenic dosages) or transplant rejection (e.g., organ transplant). In a particular embodiment, the disease is cancer. The cancer may be, without limitation: melanoma, lung cancer, lymphoma, leukemia, colon cancer, breast cancer, neuroblastoma, pancreatic cancer, prostate cancer, liver cancer, kidney cancer, brain cancer, thyroid cancer, bladder cancer, bone cancer, stomach cancer, esophageal cancer, leukemia, multiple myeloma, sarcoma, carcinoid, skin cancer, testicular cancer, ovarian cancer, pancreatic cancer, colorectal cancer, cervical cancer, endometrial cancer, or renal cancer. In a particular embodiment, the cancer forms a solid tumor. In a particular embodiment, the cancer is neuroblastoma. In a particular embodiment, the cancer is metastatic (e.g., metastatic neuroblastoma). In a particular embodiment, the cancer is non-resectable (e.g., non-resectable neuroblastoma). The methods may comprise the administration of at least one nanoparticle of the instant invention to the subject. The nanoparticles may be in a composition further comprising at least one pharmaceutically acceptable carrier.

The nanoparticles of the instant invention may be administered or used in combination with other therapies including, but not limited to, anticancer therapies (e.g., surgery (e.g., resection of the tumor or cancer cells), radiation therapy (e.g., external beam radiation, brachytherapy), and chemotherapy (e.g., administering at least one chemotherapeutic agent)), antimicrobial therapies, and immunotherapies. For example, CCL21 nanoparticles of the instant invention may be used in combination with immunotherapies such as, without limitation, antibody based therapies, checkpoint inhibitors, cell therapies including but not limited to dendritic based cell therapies and T cell based therapies, immune modulating peptides, vaccines (e.g., protein based vaccines, peptide based vaccines, and nucleic acid based vaccines), oncolytic viruses, PD1 inhibitors, or any other therapeutic that modulates the immune response. It is envisioned that CCL21 nanoparticles can be administered at the same time as other therapies, or CCL21 nanoparticles can be administered before the administration of other therapies, or CCL21 nanoparticles could be administered after the administration of other therapies. The CCL21 nanoparticles will enhance the efficacy of other administered therapies such as immunotherapies.

Chemotherapeutic agents are compounds that exhibit anticancer activity and/or are detrimental to a cell (e.g., a toxin). Suitable chemotherapeutic agents include, but are not limited to: toxins (e.g., saporin, ricin, abrin, ethidium bromide, diptheria toxin, Pseudomonas exotoxin); alkylating agents (e.g., nitrogen mustards such as chlorambucil, cyclophosphamide, isofamide, mechlorethamine, melphalan, and uracil mustard; aziridines such as thiotepa; methanesulphonate esters such as busulfan; nitroso ureas such as carmustine, lomustine, and streptozocin; platinum complexes such as cisplatin and carboplatin; bioreductive alkylators such as mitomycin, procarbazine, dacarbazine and altretamine); DNA strand-breakage agents (e.g., bleomycin); topoisomerase II inhibitors (e.g., amsacrine, dactinomycin, daunorubicin, idarubicin, mitoxantrone, doxorubicin, etoposide, and teniposide); DNA minor groove binding agents (e.g., plicamydin); antimetabolites (e.g., folate antagonists such as methotrexate and trimetrexate; pyrimidine antagonists such as fluorouracil, fluorodeoxyuridine, CB3717, azacitidine, cytarabine, and floxuridine; purine antagonists such as mercaptopurine, 6-thioguanine, fludarabine, pentostatin; asparginase; and ribonucleotide reductase inhibitors such as hydroxyurea); tubulin interactive agents (e.g., vincristine, vinblastine, and paclitaxel (Taxol®)); hormonal agents (e.g., estrogens; conjugated estrogens; ethinyl estradiol; diethylstilbesterol; chlortrianisen; idenestrol; progestins such as hydroxyprogesterone caproate, medroxyprogesterone, and megestrol; and androgens such as testosterone, testosterone propionate, fluoxymesterone, and methyltestosterone); adrenal corticosteroids (e.g., prednisone, dexamethasone, methylprednisolone, and prednisolone); leutinizing hormone releasing agents or gonadotropin-releasing hormone antagonists (e.g., leuprolide acetate and goserelin acetate); HSP90 inhibitors (e.g., 17-AAG); and antihormonal antigens (e.g., tamoxifen, antiandrogen agents such as flutamide; and antiadrenal agents such as mitotane and aminoglutethimide).

The nanoparticles or compositions of the instant invention can be administered to an animal, in particular a mammal, more particularly a human, in order to treat/inhibit/prevent the disease or disorder. The pharmaceutical compositions of the instant invention may also comprise at least one other bioactive agent, particularly at least one other therapeutic agent. The additional bioactive agent may also be administered in separate composition from the nanoparticles of the instant invention. The compositions may be administered at the same time or at different times (e.g., sequentially). When the disease is cancer, the compositions of the instant invention may also be administered with a chemotherapy (e.g., sequentially).

The nanoparticles described herein will generally be administered to a patient or subject as a pharmaceutical preparation. The term "patient" as used herein refers to human or animal subjects. These nanoparticles may be employed therapeutically, under the guidance of a physician.

The compositions comprising the nanoparticles of the instant invention may be conveniently formulated for administration with any pharmaceutically acceptable carrier(s). For example, the nanoparticles may be formulated with an acceptable medium such as water, buffered saline, ethanol, polyol (for example, glycerol, propylene glycol, liquid polyethylene glycol and the like), dimethyl sulfoxide (DMSO), oils, detergents, suspending agents or suitable mixtures thereof. The concentration of the nanoparticles in the chosen medium may be varied and the medium may be chosen based on the desired route of administration of the pharmaceutical preparation. Except insofar as any conventional media or agent is incompatible with the nanoparticles to be administered, its use in the pharmaceutical preparation is contemplated.

The dose and dosage regimen of nanoparticles according to the invention that are suitable for administration to a particular patient may be determined by a physician considering the patient's age, sex, weight, general medical condition, and the specific condition for which the nanoparticles are being administered and the severity thereof. The physician may also take into account the route of administration, the pharmaceutical carrier, and the biological activity of the nanoparticles.

Selection of a suitable pharmaceutical preparation will also depend upon the mode of administration chosen. For example, the nanoparticles of the invention may be administered by direct injection (e.g., intratumor or to the surrounding area) or intravenously. In this instance, a pharmaceutical preparation comprises the nanoparticles dispersed in a medium that is compatible with the site of injection.

Nanoparticles of the instant invention may be administered by any method. For example, the nanoparticles of the instant invention can be administered, without limitation parenterally, intratumorally, subcutaneously, orally, topically, pulmonarily, rectally, vaginally, intravenously, intraperitoneally, intrathecally, intracerbrally, epidurally, intramuscularly, intradermally, or intracarotidly. In a particular embodiment, the nanoparticles are administered by injection. In a particular embodiment, the nanoparticles are administered intratumorally, intravenously, or intraperitoneally. In a particular embodiment, the nanoparticles are administered by endoscopic injection. Pharmaceutical preparations for injection are known in the art. If injection is selected as the method for administering the nanoparticles, steps should be taken to ensure that sufficient amounts of the molecules reach their target cells to exert a biological effect. Dosage forms for parenteral administration include, without limitation, solutions, emulsions, suspensions, dispersions and powders/granules for reconstitution.

Pharmaceutical compositions containing the nanoparticles of the present invention as the active ingredient in intimate admixture with a pharmaceutically acceptable carrier can be prepared according to conventional pharmaceutical compounding techniques. The carrier may take a wide variety of forms depending on the form of preparation desired for administration, e.g., intravenous, oral, direct injection, intracranial, and intravitreal.

A pharmaceutical preparation of the invention may be formulated in dosage unit form for ease of administration and uniformity of dosage. Dosage unit form, as used herein, refers to a physically discrete unit of the pharmaceutical preparation appropriate for the patient undergoing treatment. Each dosage should contain a quantity of active ingredient calculated to produce the desired effect in association with the selected pharmaceutical carrier. Procedures for determining the appropriate dosage unit are well known to those skilled in the art.

Dosage units may be proportionately increased or decreased based on the weight of the patient. Appropriate concentrations for alleviation of a particular pathological condition may be determined by dosage concentration curve calculations, as known in the art. The dosage ranges for the administration of the compositions of the invention are those large enough to produce the desired effect. The dosage should not be so large as to cause adverse side effects, such as unwanted cross-reactions, anaphylactic reactions, and the like. Generally, the dosage will vary with the age, condition, sex and extent of the disease in the patient and can be determined by one of skill in the art. The dosage can be adjusted by the individual physician in the event of any counter indications.

In accordance with the present invention, the appropriate dosage unit for the administration of nanoparticles may be determined by evaluating the toxicity of the molecules or cells in animal models. Various concentrations of nanoparticles in pharmaceutical preparations may be administered to mice, and the minimal and maximal dosages may be determined based on the beneficial results and side effects observed as a result of the treatment. Appropriate dosage unit may also be determined by assessing the efficacy of the nanoparticles treatment in combination with other standard drugs. The dosage units of nanoparticles may be determined individually or in combination with each treatment according to the effect detected.

The pharmaceutical preparation comprising the nanoparticles may be administered at appropriate intervals, for example, at least twice a day or more until the pathological symptoms are reduced or alleviated, after which the dosage may be reduced to a maintenance level. The appropriate interval in a particular case would normally depend on the condition of the patient.

### Definitions

The following definitions are provided to facilitate an understanding of the present invention:
The singular forms "a," "an," and "the" include plural referents unless the context clearly dictates otherwise.

As used herein, the term "about" means that dimensions, sizes, formulations, parameters, shapes and other quantities and characteristics are not and need not be exact, but may be approximate and/or larger or smaller, as desired, reflecting tolerances, conversion factors, rounding off, measurement error and the like, and other factors known to those of skill in the art. In general, a dimension, size, formulation, parameter, shape or other quantity or characteristic is "about" or "approximate" whether or not expressly stated to be such. It is noted that embodiments of very different sizes, shapes and dimensions may employ the described arrangements.

The transitional terms "comprising", "consisting essentially of' and "consisting of', when used in the appended claims, in original and amended form, define the claim scope with respect to what unrecited additional claim elements or steps, if any, are excluded from the scope of the claim(s). The term "comprising" is intended to be inclusive or open-ended and does not exclude any additional, unrecited element, method, step or material. The term "consisting of' excludes any element, step or material other than those specified in the claim and, in the latter instance, impurities ordinary associated with the specified material(s). The term "consisting essentially of' limits the scope of a claim to the specified elements, steps or material(s) and those that do not materially affect the basic and novel characteristic(s) of the claimed invention. All materials, manufactures, and methods described herein that embody the present invention can, in alternate embodiments, be more specifically defined by any of the transitional terms "comprising," "consisting essentially of," and "consisting of."

"Pharmaceutically acceptable" indicates approval by a regulatory agency of the Federal or a state government or listed in the U.S. Pharmacopeia or other generally recognized pharmacopeia for use in animals, and more particularly in humans.

A "carrier" refers to, for example, a diluent, adjuvant, preservative (e.g., Thimersol, benzyl alcohol), anti-oxidant (e.g., ascorbic acid, sodium metabisulfite), solubilizer (e.g., polysorbate 80), emulsifier, buffer (e.g., Tris HCl, acetate, phosphate), antimicrobial, bulking substance (e.g., lactose, mannitol), excipient, auxiliary agent or vehicle with which an active agent of the present invention is administered. Pharmaceutically acceptable carriers can be sterile liquids, such as water and oils, including those of petroleum, animal, vegetable or synthetic origin. Water or aqueous saline solutions and aqueous dextrose and glycerol solutions may be employed as carriers, particularly for injectable solutions. Suitable pharmaceutical carriers are described in "Remington's Pharmaceutical Sciences" by E.W. Martin (Mack Publishing Co., Easton, PA); Gennaro, A. R., Remington: The Science and Practice of Pharmacy, (Lippincott, Williams and Wilkins); Liberman, et al., Eds., Pharmaceutical Dosage Forms, Marcel Decker, New York, N.Y.; and Kibbe, et al., Eds., Handbook of Pharmaceutical Excipients, American Pharmaceutical Association, Washington.

The term "isolated" may refer to a compound (e.g., nanoparticle) that has been sufficiently separated from the environment from which it was in or associated (e.g., so as to exist in "substantially pure" form). The term "isolated" is not meant to exclude artificial or synthetic mixtures with other compounds or materials, or the presence of impurities that do not interfere with the fundamental activity, and that may be present, for example, due to incomplete purification, or the addition of stabilizers.

The term "treat" as used herein refers to any type of treatment that imparts a benefit to a patient afflicted with a disease, including improvement in the condition of the patient (e.g., in one or more symptoms), delay in the progression of the condition, etc.

As used herein, the term "prevent" refers to the prophylactic treatment of a subject who is at risk of developing a condition (e.g., cancer) resulting in a decrease in the probability that the subject will develop the condition.

As used herein, the term "subject" refers to an animal, particularly a mammal, particularly a human.

A "therapeutically effective amount" of a compound or a pharmaceutical composition refers to an amount effective to prevent, inhibit, treat, or lessen the symptoms of a particular disorder or disease. The treatment of cancer herein may refer to curing, relieving, and/or preventing the cancer, the symptom(s) of it, or the predisposition towards it.

A "therapeutically effective amount" of a compound or a pharmaceutical composition refers to an amount effective to prevent, inhibit, or treat a particular disorder or disease and/or the symptoms thereof. For example, "therapeutically effective amount" may refer to an amount sufficient to modulate stress and/or stress response in a subject.

As used herein, the term "amphiphilic" means the ability to dissolve in both water and lipids/apolar environments. Typically, an amphiphilic compound comprises a hydrophilic portion and a hydrophobic portion. "Hydrophobic" designates a preference for apolar environments (e.g., a hydrophobic substance or moiety is more readily dissolved in or wetted by non-polar solvents, such as hydrocarbons, than by water). As used herein, the term "hydrophilic" means the ability to dissolve in water.

As used herein, the term "polymer" denotes molecules formed from the chemical union of two or more repeating units or monomers. The term "block copolymer" most simply refers to conjugates of at least two different polymer segments, wherein each polymer segment comprises two or more adjacent units of the same kind.

The following example provides illustrative methods of practicing the instant invention and is not intended to limit the scope of the invention in any way.

### EXAMPLE

Figure 1 provides a schematic of the nanoformulations of the instant invention involving alginate ionotropic pre-gelation in the presence of CCL21 followed by protamine sulfate polyelectrolyte complexation. An advantage of using such a hydrogel system relies on the ability of the anionic polysaccharide chains to anchor cationic CCL21 within alginate network via electrostatic interactions. The CCL21-loaded nanogels will combine several key characteristics that will ascertain a long-lasting biological function of CCL21, such as easy and efficient protein loading, nanoscale size, and protection from disintegration. These properties can be tailored by modulating the physicochemical properties of alginate copolymers (molecular mass, chemical composition), alginate/protamine sulfate ratio, and density of cross-linking as well as by the overall procedure used for their preparation. Incorporation of amphiphilic copolymers into cross-linked alginate network can further alter the porosity/permeability of the alginate nanoparticles for tailoring the release of chemokine (Fig. 1). An attractive aspect of the proposed approach is structural versatility. The potential to create a series of CCL21-loaded nanomaterials with various structural compositions provides a broad material base for the design and optimization of the chemokine nanocarrier that releases CCL21 in a manner mimicking normal secretion from cells, or, if so desired, at a rate faster or slower than normal secretion from cells.

The alginate/protamine sulfate nanoparticle production was performed using an aqueous environment, based on the ionotropic gelation of alginate with calcium chloride followed by crosslinking with protamine sulfate. Briefly, aqueous solutions of sodium alginate (0.75 mg/mL) and CCL21 (1 mg/mL or 5 mg/mL) were mixed at different proportions to give targeted drug to polymer ratios (e.g. 1/5, 1/10, 1/20, 1/40, 1/60 w/w). Calcium chloride solution was then added drop-wise upon stirring at 800 rpm for 30 minutes to provide alginate pre-gel. The alginate:CaCl₂ ratio was varied from 4:1 to 8:1 (w/w). A solution of protamine sulfate was then added drop-wise into the pre-gel followed by additional stirring for 2 hours at room temperature to improve curing. The alginate:protamine sulfate mass ratio was varied from 50:1 to 1:1 (w/w). Unbound protein was separated by centrifugation (20,000 rpm, 40 minutes). The amount of unbound CCL21 in the supernatant was assayed by HPLC. To prepare composite nanoparticles, Pluronic® F127 was added to alginate/protein solution prior to cross-linking with calcium. F127 concentration in the reaction mixtures was varied from 0.1% to 1%.

The hydrodynamic diameter and surface charge (ξ-potential) of the protein-loaded particles was determined by dynamic light scattering (DLS) and particle morphology was analyzed by transmission electron microscopy and atomic force microscopy. For the design of the release studies, the aqueous formulations containing protein (0.4 µg/mL of CCL21) in phosphate-buffered saline (PBS; pH 7.4) were placed in a dialysis bag (Spectra/Por-6 tubing, MW cut-off 50,000) and dialyzed at 37°C against 10% FBS in the same buffer. The release was monitored over the course of 5 or more days. All experiments were performed in triplicate. The amount of protein in the dialysate was determined by HPLC.

The protein loading (% wt incorporated protein/wt copolymer) and encapsulation efficiency (% wt incorporated protein/wt total added protein) were calculated. These data allow for determining 1) best ratios of nanoparticle formulation components and 2) optimal drug/polymer compositions to achieve high loading and efficiency. In addition to protein loading, the stability of the formulations over time was studied. Briefly, the stability of the particles upon storage was determined by measuring protein concentration in solution over 12, 24, 48, 72 and 240 hours at 25°C and 4°C. Furthermore, changes in size of the alginate nanoparticles were evaluated to characterize the stability of the nanoparticles in dispersion in response to the changes of temperature (25°C vs. 37°C) and lyophilization/redispersion procedure. Release data was plotted as % protein released (mean ± SD) over time.

Initial experiments were performed with cytochrome C as a model protein as it has similar physical characteristics to CCL21 (e.g., cytochrome C: 12.27 kDa, pI 10.1; CCL21: 12.1 kDa, pI 10.13). Nanoparticles comprising cyctochrome C were synthesized using a CaCl₂:alginate ratio of 0.15 (w/w) and an alginate : cytochrome C ratio of 6:1 or 60:1. The nanoparticles had a PDI of about 0.22. DLS and TEM characterization of the nanoparticles shows the nanoparticles had an average size of less than 300 nm. The addition of F127 to the synthesis process resulted in nanoparticles having a PDI of about 0.20 and a smaller average diameter. Protein release studies with the cytochrome C nanoparticles showed that the addition of F127 caused the nanoparticles to retain the protein for longer than in its absence. Further, the alginate : cytochrome C ratio of 60:1 resulted in a faster release of protein than a ratio of 6:1. In addition, the nanoparticles were also synthesized with a CaPO₄ shell, which also decreased the alginate nanoparticle size.

Nanoparticles comprising CCL21 were then synthesized. First, the effect of varying CaCl₂ concentration was studied. As seen in Figure 2A, variation in the calcium chloride concentration alters the size of the CCL21-alginate nanoparticles. Lower CaCl₂ resulted in smaller alginate particles. However, at certain low concentrations of CaCl₂, the polydispersity of the samples increased and the particles were not as thermodynamically stable. Based on these types of studies, the desired CaCl₂ concentration range was established.

Dynamic light scattering was used to determine the size distribution of the CCL21-alginate nanoparticles. As seen in Figure 2B, the CCL21 nanoparticles with F127 (0.5%) are fairly uniform and have an average diameter less than 300 nm. Figure 2C provides a transmission electron micrograph of CCL21-alginate-F127 nanoparticles recovered after ultra-centrifugation and re-dispersal in an aqueous solution. Relatively uniform spherical forms are observed. Figure 2D provides a graph of the release of alginate:CCL21 (60:1) nanoparticles with F127. CCL21 was detected by ELISA.

The activity of the synthesized CCL21 nanoparticles wastested *in vivo* against subcutaneous neuroblastoma (Neuro2a) tumors. Briefly, mice were injected subcutaneously with 1x10⁶ Neuro2a mouse neuroblastoma cells. Tumors were allowed to grow until palpable and the mice were divided into two groups with equivalent average tumor sizes, and one group was treated with CCL21 nanoformulation and the other group with empty nanoparticles. The CCL21-alginate nanoparticle formulation included CaCl₂, protamine sulfate, and F127 in addition to CCL21 and alginate, and the empty nanoparticles had all these components except CCL21. The mice were given intratumoral injections of CCL21 nanoparticles (containing 6 micrograms of CCL21) or an equivalent amount of empty nanoparticles in the morning and in the evening on each of two consecutive days. The length and width dimensions of the tumors were measured at intervals and the mice were euthanized if the tumors were at or exceeding 1 cc in volume at the time of measurement. The percentage of surviving mice at various time points is graphed on a Kaplan-Meier survival curve in Figure 3. The median survival for nanoformulation CCL21-treated mice was 12 days and the median survival for the empty control nanoparticles-treated mice was 8.5 days (p=0.0276).

In an independent experiment, equivalent amounts of CCL21 (in PBS solution or in nanoformulation) were delivered intratumorally to two groups of subcutaneous Neuro2a tumor-bearing mice. A dose of 6 micrograms of CCL21 in PBS/0.05% mouse serum or in nanoformulation was injected into the tumors in the morning and in the evening on each of two consecutive days. The CCL21-alginate nanoparticle formulation included CaCl₂, protamine sulfate, F127, CCL21, and alginate. The length and width dimensions of the tumors were measured intermittently and the tumor volumes were calculated and graphed. At Day 18, a statistical comparison of tumor volumes was performed. Figures 4A (nanoformulation of CCL21) and 4B (CCL21) provide the tumor volumes of individual mice. The mice were euthanized if the tumors were at or above 1 cc in volume at the time of measurement or if they exhibited specific signs of morbidity (e.g., tumor ulceration).

Repeated measures ANOVA models were used to look at changes in tumor size over time. The model included fixed effects for group, day and the group x day interaction and day is modeled as a continuous variable. A first-order autoregressive covariance structure was used to model the covariance structure between mice. SAS software was used for data analysis (SAS Institute Inc.). For the repeated measures analysis of tumor size, the model includes fixed effects for group, day and the group x day interaction. Day is modeled as a continuous variable. A first-order autoregressive covariance structure is used to model the covariance structure between mice. There is a significant group x day interaction (p=0.014), which indicates that changes over time are different depending on the group. Both treatment groups have tumor sizes increasing, but the nanoparticle CCL21-treated group is increasing at a significantly slower rate than the CCL21 only-treated group. On average, the nanoparticle CCL21-treated group tumor size increases 0.0319 cc/day (p=0.0002) and the CCL21 only-treated group tumor size increases 0.0692 cc/day (p=0.0001).

Survival was charted on a Kaplan-Meier curve and the log rank test was used to compare survival distributions between groups (Figure 4C). The median survival for nanoparticle CCL21-treated mice was 17.0 days and the free CCL21-treated mice was only 10.5 days (p = 0.05). Notably, four mice in the nanoparticle CCL21-treated group survived beyond 32 days and showed complete regressions/clearing of the tumor.

## Claims

1. A nanoparticle comprising alginate, C-C motif chemokine ligand 21(CCL21), protamine sulfate, and a divalent cation.

2. The nanoparticle of claim 1, wherein said divalent cation is Ca²⁺.

3. The nanoparticle of any one of the preceding claims, wherein said nanoparticle has a diameter less than or equal to 300 nm.

4. The nanoparticle of any one of the preceding claims, wherein said CCL21 is contained within a polymeric mesh comprising the alginate and protamine sulfate.

5. The nanoparticle of any one of the preceding claims, wherein said nanoparticle further comprises an amphiphilic block copolymer.

6. The nanoparticle of claim 5, wherein said amphiphilic block copolymer comprises at least one block of poly(oxyethylene) and at least one block of poly(oxypropylene).

7. The nanoparticle of claim 5, wherein said amphiphilic block copolymer is poloxamer 407.

8. The nanoparticle of any one of the preceding claims, wherein the alginate to CCL21 ratio (w/w) is about 1:1 to about 100:1 or about 6:1 to about 60:1.

9. The nanoparticle of any one of the preceding claims, wherein the alginate to protamine sulfate ratio (w/w) is about 1:1 to about 100:1, about 5:1 to about 50:1, or about 3:1 to about 30:1.

10. A composition comprising the nanoparticle of any one of the preceding claims and at least one pharmaceutically acceptable carrier.

11. The nanoparticle of any one of claims 1 to 9 for use in treating cancer, infectious disease, autoimmune disorder, or transplant rejection.

12. The nanoparticle for use of claim 11 for use in treating cancer.

13. The nanoparticle for use of claim 12, wherein said cancer is neuroblastoma or is selected from the group consisting of melanoma, lung cancer, lymphoma, leukemia, colon cancer, breast cancer, neuroblastoma, pancreatic cancer, prostate cancer, liver cancer, kidney cancer, brain cancer, thyroid cancer, bladder cancer, bone cancer, stomach cancer, esophageal cancer, leukemia, multiple myeloma, sarcoma, carcinoid, skin cancer, testicular cancer, ovarian cancer, pancreatic cancer, colorectal cancer, cervical cancer, endometrial cancer, and renal cancer.

14. A method of synthesizing a nanoparticle of any one of claims 1 to 9, said method comprising:
a) adding a salt of the divalent cation to an aqueous solution of CCL21 and alginate, and
b) adding protamine sulfate to the aqueous solution of step a) after addition of the salt, thereby generating said nanoparticles,
wherein said method optionally further comprises isolating the synthesized nanoparticles.

15. The method of claim 14, wherein the aqueous solution of step a) further comprises an amphiphilic block copolymer.

## Patentansprüche

1. Ein Nanopartikel umfassend Alginat, C-C-Motiv-Chemokinligand 21 (CCL21), Protaminsulfat und ein zweiwertiges Kation.

2. Das Nanopartikel gemäß Anspruch 1, wobei das genannte zweiwertige Kation Ca²⁺ ist.

3. Das Nanopartikel gemäß irgendeinem der voranstehenden Ansprüche, wobei das genannte Nanopartikel einen Durchmesser aufweist, der kleiner als oder gleich 300 nm ist.

4. Das Nanopartikel gemäß irgendeinem der voranstehenden Ansprüche, wobei das genannte CCL21 innerhalb eines polymerischen Netzes, das das Alginat und das Protaminsulfat umfasst, enthalten ists.

5. Das Nanopartikel gemäß irgendeinem der voranstehenden Ansprüche, wobei das genannte Nanopartikel weiterhin ein amphiphiles Block-Copolymer umfasst.

6. Das Nanopartikel gemäß Anspruch 5, wobei das genannte amphiphile Block-Copolymer wenigstens einen Block aus Poly(Oxyethylen) und wenigstens einen Block aus Poly(Oxypropylen) umfasst.

7. Das Nanopartikel gemäß Anspruch 5, wobei das genannte amphiphile Block-Copolymer Proloxamer 407 ist.

8. Das Nanopartikel gemäß irgendeinem der voranstehenden Ansprüche, wobei das Verhältnis Alginat zu CCL21 (Gew./Gew.) ungefähr 1: 1 bis ungefähr 100: 1 oder ungefähr 6: 1 bis ungefähr 60:1 ist.

9. Das Nanopartikel gemäß irgendeinem der voranstehenden Ansprüche, wobei das Verhältnis Alginat zu Protaminsulfat (Gew./Gew.) ungefähr 1: 1 bis ungefähr 100: 1, ungefähr 5: 1 bis ungefähr 50: 1 oder ungefähr 3: 1 bis ungefähr 30:1 ist.

10. Eine Zusammensetzung, die das Nanopartikel gemäß irgendeinem der voranstehenden Ansprüche und wenigstens einen pharmazeutisch akzeptablen Trägerstoff umfasst.

11. Das Nanopartikel gemäß irgendeinem der Ansprüche 1 bis 9 zur Verwendung in der Behandlung von Krebserkrankungen, Infektionskrankheiten, Autoimmunstörungen oder Transplantatabstoßungen.

12. Das Nanopartikel zur Verwendung des Anspruchs 11 zur Verwendung in der Behandlung einer Krebserkrankung.

13. Das Nanopartikel zur Verwendung des Anspruchs 12, wobei die genannte Krebserkrankung ein Neuroblastom ist oder aus der Gruppe ausgewählt ist, bestehend aus Melanomen, Lungenkrebs, Lymphomen, Leukämie, Darmkrebs, Brustkrebs, Neuroblastomen, Bauchspeicheldrüsenkrebs, Prostatakrebs, Leberkrebs, Nierenkrebs, Gehirnkrebs, Schilddrüsenkrebs, Blasenkrebs, Knochenkrebs, Magenkrebs, Speiseröhrenkrebs, Leukämie, multiplen Myelomen, Sarkomen, Karzinoiden, Hautkrebs, Hodenkrebs, Eierstockkrebs, Bauchspeicheldrüsenkrebs, kolorektalem Krebs, Gebärmutterkrebs, Gebärmutterschleimhautkrebs und Nierenkrebs.

14. Verfahren zur Herstellung eines Nanopartikels gemäß irgendeinem der Ansprüche 1 bis 9, wobei das genannte Verfahren umfasst:
a) Hinzufügen eines Salzes des zweiwertigen Kations zu einer wässerigen Lösung von CCL21 und Alginat, und
b) Hinzufügen von Protaminsulfat zu der wässerigen Lösung des Schritts a) nach dem Hinzufügen des Salzes, wodurch die genannten Nanopartikel erzeugt werden,
wobei das genannte Verfahren optional weiterhin das Isolieren der hergestellten Nanopartikel umfasst.

15. Verfahren gemäß Anspruch 14, wobei die wässerige Lösung des Schritts a) außerdem ein amphiphiles Block-Copolymer umfasst.

## Revendications

1. Nanoparticule comprenant un alginate, un ligand de chimiokine 21 à motif C-C (CCL21), un sulfate de protamine, et un cation divalent.

2. Nanoparticule selon la revendication 1, dans laquelle ledit cation divalent est Ca²⁺.

3. Nanoparticule selon l'une quelconque des revendications précédentes, dans laquelle ladite nanoparticule a un diamètre inférieur ou égal à 300 nm.

4. Nanoparticule selon l'une quelconque des revendications précédentes, dans laquelle ledit CCL21 est contenu dans un treillis en polymère comprenant l'alginate et le sulfate de protamine.

5. Nanoparticule selon l'une quelconque des revendications précédentes, dans laquelle ladite nanoparticule comprend, en outre, un copolymère en bloc amphiphile.

6. Nanoparticule selon la revendication 5, dans laquelle ledit copolymère en bloc amphiphile comprend au moins un bloc de poly(oxyéthylène) et au moins un bloc de poly(oxypropylène).

7. Nanoparticule selon la revendication 5, dans laquelle ledit copolymère en bloc amphiphile est un poloxamère 407.

8. Nanoparticule selon l'une quelconque des revendications précédentes, dans laquelle le rapport alginate / CCL21 (poids / poids) est d'environ 1 / 1 à environ 100 / 1 ou d'environ 6 / 1 à environ 60 / 1.

9. Nanoparticule selon l'une quelconque des revendications précédentes, dans laquelle le rapport alginate / sulfate de protamine (poids / poids) est d'environ 1 / 1 à environ 100 / 1, d'environ 5 / 1 à environ 50 / 1, ou d'environ 3 / 1 à environ 30 / 1.

10. Composition comprenant la nanoparticule selon l'une quelconque des revendications précédentes et au moins un véhicule acceptable du point de vue pharmaceutique.

11. Nanoparticule selon l'une quelconque des revendications 1 à 9, pour la mise en œuvre dans le traitement du cancer, des maladies infectieuses, des maladies auto-immunes, ou du rejet de greffes.

12. Nanoparticule selon la revendication 11, pour la mise en œuvre dans le traitement du cancer.

13. Nanoparticule selon la revendication 12, dans laquelle ledit cancer est un neuroblastome ou est choisi dans le groupe consistant en mélanome, cancer du poumon, lymphome, leucémie, cancer du côlon, cancer du sein, neuroblastome, cancer du pancréas, cancer de la prostate, cancer du foie, cancer du rein, cancer du cerveau, cancer de la thyroïde, cancer de la vessie, cancer des os, cancer de l'estomac, cancer de l'œsophage, leucémie, myélome multiple, sarcome, carcinoïdes, cancer de la peau, cancer des testicules, cancer des ovaires, cancer du pancréas, cancer colorectal, cancer cervical, cancer de l'endomètre, et cancer du rein.

14. Procédé de synthèse d'une nanoparticule selon l'une quelconque des revendications 1 à 9, ledit procédé comprenant :
a) l'addition d'un sel du cation divalent à une solution aqueuse de CCL21 et d'alginate, et
b) l'addition de sulfate de protamine à la solution aqueuse de l'étape a) après addition du sel, générant de ce fait lesdites nanoparticules,
dans lequel ledit procédé comprend en outre facultativement l'isolement des nanoparticules synthétisées.

15. Procédé selon la revendication 14, dans lequel la solution aqueuse de l'étape a) comprend, en outre, un copolymère en bloc amphiphile.
